# EUROPEAN PATENT APPLICATION

(11) **EP 2 745 905 A1**
(43) Date of publication of application: **25.06.2014**
(21) Application number: 12008534.5
(22) Date of filing: 21.12.2012
(51) Int. Cl.: B01D 15/40, C07C 67/08, C07C 68/08, C07C 51/47, C07C 59/08, C07C 57/13, C07C 53/08, C07C 57/15, C07C 59/245, C07C 57/145, C07C 69/68, C07C 69/40, C07C 69/14, C07C 69/60, C07C 69/675

(54) **Process for the purification of carboxylic acids by subcritical or supercritical fluid chromatography**

(71) Applicant: ThyssenKrupp Uhde GmbH, 44141 Dortmund (DE)
(72) Inventor: Dr. Christoph Lütge, 59425 Unna (DE)

(57) **Abstract**

The invention relates to a process for the purification of carboxylic acid comprising the steps of:
a) the carboxylic acid is transformed to its monoester and/or its diester, and
b) the mono- and/or the diester of the carboxylic acid is processed by subritical or supercritical fluid chromatography using a subcritical or supercritical mobile phase.

## Description

The invention relates to a process for the purification of carboxylic acid. Also a device for the execution of the process and a use for the process according to the invention are taught. The isolation of carboxylic acids, which are not able to be separated using distillation or which can only be separated with difficulty, is extremely complex.

Key to the industrial application of carboxylic acids, which are produced using various microorganisms in the fermentation of carbohydrate-containing substrates, is the economic viability and efficiency of separating and purifying the desired carboxylic acid from these aqueous fermentation solutions which, as well as containing carboxylic acid or carboxylic acid salts, also contain other organic acids, other by-products of fermentation, microorganisms and their components, as well as residues of the substrates, such as sugar. These impurities interfere with the subsequent processing of the carboxylic acids produced. As an example, lactic acid is polymerised to form polylactic acid which is used to produce biodegradable plastics. Extremely pure monomer must be used for this purpose to ensure that a high degree of polymerisation is achieved for the lactic acid. This has been known for some time and covered, for example, in J. Dahlmann et al, British Polymer Journal, Vol. 23 (1990), Page 235. 240.

Succinic acid, for example, is known to be similar. The qualities of the succinic acid produced can be differentiated by classifying them in terms of technical quality with a succinic acid content of at least 97 Ma-% and with a succinic acid which is specially suited for use in polymerisation (polymer grade) with a content of at least 99.5 Ma-%.

A number of patents describe the production of succinic acid from fermentation solutions, including
- extractive processes using extracting agents, such as tributylamines, trialkylamines, olefins, various alcohols, and aromatic hydrocarbons,
- processes using calcium hydroxide and sulphuric acid, wherein gypsum is produced as the by-product,
- processes using electrodialysis,
- thermal methods, such as fractional distillation or thermally staged chromatography,
- high-pressure extraction using CO₂ ,
- membrane processes, such as reverse osmosis and other filtration processes
wherein the coupling of these processes and the supplementing of them with further steps in line with prior art are discussed. These types of processes are described, amongst others, in patent specifications DE 69821951 T2 ; DE 69015233 T2 ; DE 69015019 T2 ; DE 69006555 T2 ; DE 69015019 ; DE19939690C2; DE 60028958T2 ; DE 10 2004 026152 A1.

WO2011082378A2 relates to a process for the purification of succinic acid from a fermentation broth containing ammonium succinate. The process for the purification of succinic acid described in this invention involves the use of ion exchange resins for splitting the ammonium succinate in the fermentation broth. During the passage of the fermentation broth through a cationic ion exchange resin, the ammonium succinate is split into ammonium cation and the succinate anion. The proton on the resin surface is exchanged for the ammonium ions and the succinate anion is reduced to succinic acid with the protons released from the ion exchange resin. The bound ammonium is released from the resin with the addition of a strong acid such as sulfuric acid and thereby the ion exchange resin is regenerated for subsequent use. The ammonium sulfate by-product resulting from the regeneration step of this process can be used as a source of fertilizer. This process for the separation of succinic acid from the fermentation broth containing ammonium succinate can also be carried out with an anionic ion exchange resin wherein the succinate anion is retained on the surface of the ion exchange resin and subsequently released from the ion exchange resin during the regeneration step.

Furthermore a multitude of methods pertaining to the purification of lactic acid are known.

As an example, several patents teach that distillation be used for the purification of lactic acid from aqueous solutions. EP 0986532 B2 avails itself of this type of process. DE 10 2007 045 701 B3 reveals a combined extraction with linear n-trioctylamine (TOA) and distillation. Other possibilities known from specialist literature are electrodialysis and/or esterification with an alcohol, whereby, in this case too, distillation and then hydrolysis are carried out on the ester formed. These processes are extremely expensive. In addition, a disadvantage of distillation is that part of the carbohydrate is always extracted as well which leads to a deterioration in the yield for the whole process and makes it more difficult to isolate the product.

Processes using calcium hydroxide and sulphuric acid are also known, wherein gypsum is produced in large quantities as a by-product. In this context it was also found that the lactic acid from a fermentation broth acidified with sulphuric acid, for example, which alongside free lactic acid still contains ammonium and sulphate ions, can be isolated using chromatographic methods. DE 69815369 T2 describes, for example, amongst others the separation of lactic acid from aqueous mixtures by means of adsorption into a solid adsorbent, preference being given here to the use of a solid adsorbent which adsorbs lactic acid versus lactate. According to the above-mentioned specification, weak anion exchangers in particular can be considered for isolating lactic acid. Furthermore, DE 10 2009 019 248 A1 describes chromatographic methods for the purification of organic acids, particularly for lactic acid, wherein a simulated moving bed chromatography is carried out.

The disadvantage with many processes is that additional substances are added to the process which may not be contained in the target product, and/or any traces of them in the target product can lead to restrictions in the quality and application of the product. Moreover, to some extent the practical execution of the process also involves considerable technical investment and energy consumption.

The objective of the invention is to make a process available for separating and purifying carboxylic acids from fermentation broths, the process avoiding the known disadvantages of other processes. The desired process should also supply an apparatus for carrying out the related process and a use for the current process.

The invention claims especially a process for the purification of carboxylic acid comprising the steps of:
a. the carboxylic acid is transformed to its monoester and/or its diester, and
b. the mono- and/or the diester of the carboxylic acid is processed by subcritical or supercritical fluid chromatography using a subcritical or supercritical mobile phase.

Subcritical or supercritical fluid chromatography (SFC) is a separation process where mostly carbon dioxide below or above critical pressure and temperature is used as mobile phase. Mobile phase carries sample through the column, which is packed with stationary phase. Separation of several compounds is possible due to different adsorption potentials of compounds, meaning different interaction between compound molecules and surface of stationary phase. Interactions are presented as dipole, induced dipole or as H-bond. Adsorption potential is energy required for breaking interactions between compounds and surface of stationary phase. Therefore, solvent strength of mobile phase (solubility of compounds in mobile phase) has to be large enough in order to prevent long term adsorption. Solvent strength and other properties of carbon dioxide could be manipulated with pressure and temperature changing.

In case of separation of polar compounds pure non-polar carbon dioxide should be modified with polar modifiers such as ethanol and methanol, to enhance solvent strength.

Advantages of SFC are use of compressible fluids, which are gaseous at atmospheric pressure; therefore products do not contain any solvent residues. Solubility of compounds in subcritical or supercritical fluids could be adjusted with altering density and vapor pressure, meaning changing pressure and temperature.

In order to perform process step a) synthesis of esters are well known in the state of the art and described for example in US1400852. Also some recent publications describe the transformation of succinic acid to esters as purification process (Orjuela, A., Abraham J., Yanez, A. J., Peereboom, L., Lira, C.T., Miller, D.J., A novel process for recovery of fermentation-derived succinic acid, Separation and Purification Technology, (2011), 83, 31-37. and Orjuela, A., Kolah, A., Hong, X., Lira, C. T., Miller, D. J. Diethyl succinate synthesis by reactive distillation, Separation and Purification Technology, (2012), 88, 151-162.).

Also US6291708B1 describes a process for producing an organic acid and optionally for simultaneously producing an ester of the organic acid is disclosed. The process comprises the steps of: (a) combining an aqueous diluent, an ammonium salt of an organic acid, and an alcohol, thereby forming a homogeneous liquid feed mixture; (b) rapidly heating the feed mixture at a pressure sufficient to suppress at least some vaporization of the alcohol and holding it at a temperature and for a time sufficient to decompose the ammonium salt of the organic acid into ammonia and free organic acid while rapidly removing the ammonia from the reaction-mass transfer equipment, and optionally to react at least some of the free organic acid with the alcohol to form an ester of the organic acid, thereby producing (i) a vapor product stream that comprises ammonia, water, and alcohol, and (ii) a liquid product stream that comprises free organic acid, optionally ester, and alcohol, where of the total quantity of alcohol in the vapor product stream and the liquid product stream, at least about 10% by weight is present in the liquid product stream; and (c) recovering the free organic acid and optionally the ester from the liquid product stream. The liquid feed mixture can comprise a concentrated crude or partially purified broth produced by a fermentation process.

In a preferred embodiment of the invention process step b) is performed in a pressure range from 1 bar to 1000 bar, and is preferably performed in a pressure range from 10 bar to 500 bar. The process is performed in a temperature range from 0°C to 200°C, and is preferably performed in a temperature range from 5°C to 80°C.

The stationary phase of the subcritical or supercritical fluid chromatography is performed at a chiral or an achiral stationary phase. In an embodiment of the inventive process the achiral stationary phase is based on silica dioxide. To the stationary phase functional groups are attached. The functional groups are selected from a group comprising hydroxyl groups, fluorophenyl functional groups, cyano groups, amino functional groups, amide functional groups, chains of hydrocarbons with 1, 6, 8 or 18 carbon atoms, phenyl functional groups, molecules of glycerol reacted with silanol groups and chemically bonded 2-ethylpyridine. The functional groups are especially selected from a group comprising 2- and 4-ethylpyridine, dipyridyl, dicyanoimidazole, morpholine, propylacetamide, benzamide, methanesulfonamide, benzenesulfonamide, 4-fluorobenenesulfonamide, 4-nitrobenenesulfonamide, diethylaminopropyl and 3-aminopropyl-N-dinitrotoluene.

A number of silca-based amide, urea, sulfonamide and pyridine containing stationary phases are commercially available.

In a further embodiment the chiral stationary phase is based on oligosaccharides selected from the group comprising cellulose or cyclodextrin. For these columns CHIRSLPAK IA (derivative of amylase), CHIRALPAK IB and CHIRALPAK IC (derivative of cellulose), CHIRALPAK AD^{®} and CHIRALPAK AS^{®} CHIRALCEL OD and OJ, Ion exchange columns and ligand exchange columns can be used.

Furthermore process step b) is performed by selecting the subcritical or supercritical mobile phase from a group comprising carbon dioxide, nitrous oxide, propane, sulphur hexafluoride, ethane and mixtures thereof.

The carboxylic acid to be purified is selected from a group comprising lactic acid, succinic acid, acetic acid, fumaric acid, malic acid and maleic acid.

In case the carboxylic acid is desired the mono- and/or the diester of the carboxylic acid is hydrolyzed back to the carboxylic acid by methods known in the state of the art.

The patent application also relates to a device for carrying out the mentioned process. The patented apparatus typically comprises a device for producing urea granulates **characterized in that** the device comprises
- means for transformation of carbonic acid into its mono and/or its diesters
- means for processing the mono and/or diester of carbonic acid by subcritical or supercritical fluid chromatography

In an embodiment of the apparatus the means for subcritical or supercritical fluid chromatography have a chiral or an achiral stationary phase. Preferably the achiral stationary phase is based on silica dioxide. To the stationary phase functional groups are attached. The functional groups are selected from a group comprising hydroxyl groups, fluorophenyl functional, cyano groups, amino functional groups, amide functional groups, chains of hydrocarbons with 1, 6, 8 or 18 carbon atoms, phenyl functional groups, molecules of glycerol reacted with silanol groups and chemically vonded 2-ethylpyridine. The functional groups are especially selected from a group comprising 2- and 4-ethylpyridine, dipyridyl, dicyanoimidazole, morpholine, propylacetamide, benzamide, methanesulfonamide, benzenesulfonamide, 4-fluorobenenesulfonamide, 4-nitrobenenesulfonamide, diethylaminopropyl and 3-aminopropyl-N-dinitrotoluene.

In a further embodiment the chiral stationary phase is based on oligosaccharides selected from the group comprising cellulose or cyclodextrin.

Further on, the subcritical or supercritical chromatography comprises a detector selected from the group comprising a UV-VIS spectrophotometric detector, diode array UV detector, infrared spectrophotometric detector with high pressure cell, flame ionization detector and evaporative light scattering detectors.

The subcritical or supercritical chromatography comprises also in an embodiment of the current invention a mass spectrometer.

The current invention also claims a use of the inventive process in that carboxylic acids of a purity of 97 - 99.9 % are generated.

In the following the current invention is described by way of example.

### Example 1:

A solid mixture of succinic acid and maleic acids was transfered to monomethyl and dimethyl esters of acids. Chromatograpic separation was performed on pilot scale unit operated at 320 bar and temperature 40°C and is equipped with FID detector (temperature 275°C). The mobile phase was pure carbon dioxide and the flow rate was 150kg/h. As a stationary phase the Lichrospherer 100, Chromsep, RP8 with dimension 800mm×100mm, particle size 5µm was used. The separation of monomethyl esters as well of dimethyl esters was very efficient and the products with purity of 99.1 % could be obtained.

### Example 2:

A solid mixture of succinic acid and lactic acids was transfered to monomethyl and dimethyl esters of their acids. Chromatograpic separation was performed on pilot scale unit operated at 300 bar and temperature 60°C and is equipped with FID detector (temperature 275°C). The mobile phase was pure carbon dioxide and the flow rate was 200 kg/h. As a stationary phase the Lichrospherer 100, Chromsep, RP8 with dimension 850mm×100mm, particle size 5µm was used. The separation of monomethyl esters as well of dimethyl esters was very efficient and the products with purity of 98.9% could be obtained.

### Example 3:

A solid mixture of succinic acid and pyruvic acids was transfered to monomethyl and dimethyl esters of acids. Chromatograpic separation was performed on pilot scale unit operated at 310 bar and temperature 55°C and is equipped with FID detector (temperature 275°C). The mobile phase was pure carbon dioxide and the flow rate was 150kg/h. As a stationary phase the Lichrospherer 100, Chromsep, RP8 with dimension 800mm×100mm, particle size 5µm was used. The separation of monomethyl esters as well of dimethyl esters was very efficient and the products with purity of 99.7% could be obtained.

### Example 4:

A solid mixture of succinic acid and maleic acid was transfered to monomethyl and dimethyl esters of acids. Chromatograpic separation was performed on pilot scale unit operated at 150 bar and temperature 40°C and is equipped with FID detector (temperature 275°C). The mobile phase was pure propan and the flow rate was 80kg/h. As a stationary phase the Lichrospherer 100, Chromsep, RP8 with dimension 800mm×100mm, particle size 5µm was used. The separation of monomethyl esters as well of dimethyl esters was very efficient and the products with purity of 98.7% could be obtained.

### Example 5:

A solid mixture of succinic acid and maleic acid was transfered to monomethyl and dimethyl esters of acids. Chromatograpic separation was performed on pilot scale unit operated at 290 bar and temperature 80°C and is equipped with ELSD detector. The mobile phase was pure carbon dioxide and the flow rate was 150kg/h. As a stationary phase the CHIRALCEL OD with dimension 830mm×100mm, particle size 10µm was used. The separation of monomethyl esters as well of dimethyl esters was very efficient and the products with purity of 99.3% could be obtained.

### Example 6:

A solid mixture of succinic acid and pyruvic acids was transfered to monomethyl and dimethyl esters of acids. Chromatograpic separation was performed on pilot scale unit operated at 820 bar and temperature 110°C and is equipped with MS detector. The mobile phase was mixture propane/carbon dioxide (mass ratio1:1) and the flow rate was 200kg/h. As a stationary phase the CHIRALCEL OD with dimension 830mm×100mm, particle size 10µm was used. The separation of monomethyl esters as well of dimethyl esters was very efficient and the products with purity of 99.0% could be obtained.

### Example 7:

A mixture of lactic acid and acetic acids was transfered to monomethyl and dimethyl esters of acids. Chromatograpic separation was performed on pilot scale unit operated at 280 bar and temperature 20°C and is equipped with ELSD detector. The mobile phase was pure sulfurhexafluoride and the flow rate was 195kg/h. As a stationary phase the CHIRALCEL OD with dimension 560mm×100mm, particle size 10µm was used. The separation of monomethyl esters as well of dimethyl esters was very efficient and the products with purity of 99.2% could be obtained.

### Example 8:

A mixture of lactic acid and maleic acids was transfered to monomethyl and dimethyl esters of acids. Chromatograpic separation was performed on pilot scale unit operated at 423 bar and temperature 60°C and is equipped with ELSD detector. The mobile phase was pure sulfurhexafluoride and the flow rate was 295kg/h. As a stationary phase the silica modified with 2- and 4-Ethylpyridine with dimension 670m×100mm, particle size 5µm was used. The separation of monomethyl esters as well of dimethyl esters was very efficient and the products with purity of 99.0% could be obtained.

### Example 9:

A mixture of lactic acid and succinic acids was transfered to monomethyl and dimethyl esters of acids. Chromatograpic separation was performed on pilot scale unit operated at 370 bar and temperature 95°C and is equipped with ELSD detector. The mobile phase was pure carbone dioxide and the flow rate was 195kg/h. As a stationary phase the silica modified with 2- and 4-Ethylpyridine with dimension 600mm×100mm, particle size 5µm was used. The separation of monomethyl esters as well of dimethyl esters was very efficient and the products with purity of 96.8% could be obtained.

### Example 10:

A mixture of lactic acid and succinic acids was transfered to monomethyl and dimethyl esters of acids. Chromatograpic separation was performed on pilot scale unit operated at 277 bar and temperature 35°C and is equipped with MS detector. The mobile phase was pure carbone dioxide and the flow rate was 195kg/h. As a stationary phase the silica modified with fluorophenyl functional groups with dimension 700mm×100mm, particle size 5µm was used. The separation of monomethyl esters as well of dimethyl esters was very efficient and the products with purity of 97.9% could be obtained.

## Claims

1. Process for the purification of carboxylic acid comprising the steps of:
a) the carbocylic acid is transformed to its monoester and/or its diester, and
b) the mono- and/or the diester of the carboxylic acid is processed by subritical or supercritical fluid chromatography using a subcritical or supercritical mobile phase.

2. Process according to claim 1, **characterized in that** process step b) is performed in a pressure range from 1 bar to 1000 bar, and is preferably performed in a pressure range from 10 bar to 500 bar.

3. Process according to any preceding claim, **characterized in that** process step b) is performed in a temperature range from 0°C to 200°C, and is preferably performed in a temperature range form 5°C to 80°C.

4. Process according to any preceding claim, **characterized in that** process step b) is performed at a chiral or an achiral stationary phase.

5. Process according to claim 4, **characterized in that** the achiral stationary phase is based on silica dioxide.

6. Process according to claim 5, **characterized in that** functional groups are attached to the stationary phase, which functional groups are selected from a group comprising hydroxyl groups, fluorophenyl functional groups, cyano groups, amino functional groups, amide functional groups, chains of hydrocarbons with 1, 6, 8 or 18 carbon atoms, phenyl functional groups, molecules of glycerol reacted with silanol groups and chemically bonded 2-ethylpyridine.

7. Process according to claim 6, **characterized in that** the functional groups are selected from a group comprising 2- and 4-ethylpyridine, dipyridyl, dicyanoimidazole, morpholine, propylacetamide, benzamide, methanesulfonamide, benzenesulfonamide, 4-fluorobenenesulfonamide, 4-nitrobenenesulfonamide, diethylaminopropyl and 3-aminopropyl-N-dinitrotoluene.

8. Process according to claim 4, **characterized in that** the chiral stationary phase is based on oligosaccharides selected from the group comprising cellulose or cyclodextrin.

9. Process according to any preceding claim, **characterized in that** process step b) is performed by selecting the subcritical or supercritical mobile phase from a group comprising carbon dioxide, nitrous oxide, propane, sulphur hexafluoride, ethane and mixtures thereof.

10. Process according to any preceding claim, **characterized in that** the carboxylic acid to be purified is selected from a group comprising lactic acid, succinci acid, acetic acid, fumarid acid, malic acid and maleic acid.

11. Apparatus for the purification of carboxylic acid comprising
- means for transformation of carbonic acid into its mono and/or its diesters
- means for processing the mono and/or diester of carbonic acid by supercritical fluid chromatography

12. Apparatus according to claim 11, **characterized in that** means for subcritical or supercritical fluid chromatography have a chiral or an achiral stationary phase.

13. Apparatus according to claim 12, **characterized in that** the achiral stationary phase is based on silica dioxide.

14. Apparatus according to claim 13, **characterized in that** functional groups are attached to the stationary phase, which functional groups are selected from a group comprising hydroxyl groups, fluorophenyl functional, cyano groups, amino functional groups, amide functional groups, chains of hydrocarbons with 1, 6, 8 or 18 carbon atoms, phenyl functional groups, molecules of glycerol reacted with silanol groups and chemically vonded 2-ethylpyridine.

15. Apparatus according to claim 14, **characterized in that** the functional groups are selected from a group comprising 2- and 4-ethylpyridine, dipyridyl, dicyanoimidazole, morpholine, propylacetamide, benzamide, methanesulfonamide, benzenesulfonamide, 4-fluorobenenesulfonamide, 4-nitrobenenesulfonamide, diethylaminopropyl and 3-aminopropyl-N-dinitrotoluene.

16. Apparatus according to claim 13, **characterized in that** the chiral stationary phase is based on oligosaccharides selected from the group comprising cellulose or cyclodextrin.

17. Apparatus according to any of claims 11 to 16, **characterized in that** the means for subcritical or supercritical chromatography comprises a detector selected from the group comprising a UV-VIS spectrophotometric detector, diode array UV detector, infrared spectrophotometric detector with high pressure cell, flame ionization detector and evaporative light scattering detectors.

18. Apparatus according to any of claims 11 to 17, **characterized in that** the means for subcritical or supercritical chromatography comprises a mass spectrometer.

19. Use of the process according to claim 1, **characterized in that** carboxylic acids of a purity of 97 - 99.9 % are generated.
